Europäisches Patentamt

European Patent Office

Office européen des brevets

(11) Publication number: **0 108 817**
**A1**

(12) # EUROPEAN PATENT APPLICATION

(21) Application number: 82110230.8

(51) Int. Cl.³: **A 61 K 31/70**

(22) Date of filing: 06.11.82

(43) Date of publication of application: **23.05.84**
**Bulletin 84/21**

(84) Designated Contracting States: **AT BE CH DE FR GB IT LI LU NL SE**

(71) Applicant: **Kanegafuchi Chemical Industry Co., Ltd., 2-4 Nakanoshima 3-chome, Kita-ku Osaka-shi Osaka-fu (JP)**

(72) Inventor: **Kozaki, Yuichi, No. 11A-102, 684-33, Yamanoue Hiraoka-cho, Kakogawa-city Hyogo prefecture (JP)**
Inventor: **Nabekura, Keiko, No. 17-14, Nishihata 1-chome, Takasago city Hyogo prefecture (JP)**
Inventor: **Kawaharada, Hajime, No. 2183-4, Shinzaike Hiraoka-cho, Kakogawa city Hyogo prefecture (JP)**
Inventor: **Watanabe, Kiyoshi, No. 15-41, Matsugaoka 5-chome, Akashi city Hyogo prefecture (JP)**

(74) Representative: **Türk, Dietmar, Dr. rer. nat. et al, Redies, Redies, Türk & Gille Patentanwälte Brucknerstrasse 20, D-4000 Düsseldorf 13 (DE)**

(54) Stable composition of S-adenosyl-L-methionine and process for preparation thereof.

(57) A stable composition of S-adenosyl-L-methionine is disclosed which includes a salt of S-adenosyl-L-methionine and lactose and/or maltose.

The salt of S-adenosyl-L-methionine is, for example, a salt of S-adenosyl-L-methionine with hydrobromic acid, hydrochloric acid, hydroiodic acid, sulfuric acid, or p-toluenesulfonic acid; or a double salt of S-adenosyl-L-methionine with the foregoing acids. Lactose and/or maltose in the composition are preferably anhydrous.

A process for preparation of the composition is also disclosed.

The composition is suitable for preparing pharmaceutical preparations of S-adenosyl-L-methionine.

Stable Composition of S-adenosyl-L-methionine

and Process for preparation thereof

### BRIEF SUMMARY OF THE INVENTION

The present invention relates to a stable composition of S-adenosyl-L-methionine (hereinafter referred to as SAM). More specifically, the present invention relates to a stable composition of SAM comprising a salt of SAM and lactose and/or maltose. The present invention also relates to a process for preparation of the SAM composition. The composition of the present invention is suitable for pharmaceutical uses.

It is known that SAM occurs widely in living organisms and acts as a methyl donor in many transmethylations in vivo. Pharmaceutical effects of SAM have been reported in various disorders such as adipohepatica, hyperlipemia, arteriosclerosis, depression, arthritis deformans, pains in some neurological manifestations, and sleeplessness.

Since SAM, as it is, is too unstable for pharmaceutical uses, many salts and compositions of SAM have been proposed, for example SAM·p-toluenesulfonate (German patent first publication No. 2336401, Japanese patent publication No. 35726/1977) a double salt of SAM with p-toluenesulfonic acid and sulfuric acid (German patent first publication No.2430999, Japanese patent publication No.35727/1977); SAM·methanesulfonate, SAM·ethanesulfonate, SAM·dodecanesulfonate, SAM·1-octadecanesulfonate, SAM·2-chloroethane-sulfonate, SAM·2-bromoethanesulfonate, SAM·2-oxyethanesulfonate, SAM·3-oxypropanesulfonate, SAM·d,1-10-camphorsulfonate, SAM·d,1-3-bromocamphorsulfonate, SAM·cystenate, SAM·benzenesulfonate, SAM·p-chlorobenzenesulfonate, SAM·2-mesitylbenzenesulfonate, SAM·4-biphenylsulfonate, SAM·1-naphthalenesulfonate, SAM·2-naphthalenesulfonate, SAM·5-sulfosalicylate, SAM·p-acetylbenzenesulfonate, SAM·1,2-ethanedisulfonate, SAM·o-benzenedisulfonate, SAM·chondroitinsulfate, and a double salt of SAM with sulfuric acid and said sulfonic acids or chondroitinsulfate (German patent first publication No.2530898, Japanese patent first publication No.125717/1976); SAM·1,5-naphthalenesulfonate and SAM·1-amino-8-naphthol-2,4-disulfonate, (Japanese patent first publication No.38614/1978); SAM·citrate, SAM·tartrate, SAM·maleate, and SAM·ascorbate (French patent No.2275220); a composition comprising SAM, sulfuric acid, and ribonucleotide-5'-monosulfate (Japanese patent first publication No. 109998/1979); a composition comprising SAM, sulfuric acid, and nucleotidesulfate (Japanese patent first publication No. 154774/1979); a composition comprising SAM, sulfuric acid, and a sulfate of monosaccharide or oligosaccharide (Japanese patent first publication No.105700/1980); a composition comprising SAM sulfuric acid, and cytidine-5'-monosulfate (Japanese patent first publication No.28808/1979); a composition comprising SAM, sulfuric acid, and uridine-2'(3'),5'-disulfate (Japanese patent first publication No.55598/1979): and a composition comprising SAM or its salt and a lithium salt (Japanese patent first publication No.125194/1977).

Known SAM preparations, however, are not sufficiently stable but are irritant and/or toxic as pharmaceuticals. Moreover, many of known SAM preparations require complicated and costly puocedures for their preparation.

Thus a sufficiently stable composition of SAM which can be prepared economically and is suitable for pharmaceutical uses has not been known; therefore, such a preparation of SAM has been looked forward to.

The present inventors have undertaken various investigations in search of a stable composition of SAM which can be prepared economically and is suitable for pharmaceutical uses and, as a result, have now completed the present invention.

The composition of SAM of the present invention comprises a salt of SAM and lactose and/or maltose.

The salt of SAM in the composition of the present invention is a salt of SAM with an inorganic or organic acid. It also includes a double salt of SAM with inorganic or organic acids. Examples of the salt of SAM are those with hydrobromic acid, hydrochloric acid, hydroiodic acid, sulfuric acid, and p-toluenesulfonic acid; or double salts of SAM with the foregoing acids.

The lactose and maltose in the composition of the present invention are preferably anhydrous.

One of the processes for preparation of the composition of the present invention comprises dissolving the salt of SAM described above and lactose and/or maltose in an aqueous solvent to prepare an aqueous solution of said two components and then lyophilizing the aqueous solution. Another method for preparation of the composition of the present invention comprises mixing a salt of SAM with lactose and/or maltose in powdery form.

According to the present invention, a composition of SAM which is stable for a long period of time even at a room temperature and which can be prepared readily at low cost is provided for pharmaceutical uses.

The composition of SAM of the present invention can be blended with suitable pharmaceutically acceptable excipients and additives to provide pharmaceutical preparations, in particular, stable oral preparations of SAM, such as tablets, pills, and capsules of SAM, and a stable injection of SAM.

-4-

DETAILED DESCRIPTION

The salt of SAM in the composition of the present invention can be obtained, for example, by the following known methods;

A.    Preparation of SAM

1.    SAM is produced in cells of microorganisms when the microorganisms are cultivated in a medium containing methionine [F. Schlenk et al., J. Biol. Chem., 229, 1051 (1957); F Schlenk et al., Enzymologia, 29, 283 (1965)]. The microorganisms include, for example, those belonging to the genus Saccharomyces, Candida, Hansenula, Pichia, Cryptococcus, Rhodotorula, Trichosporon, Kloeckera, Torulopsis,Hanseniaspora, Sporobolomyces, Lipomyces, Tolula, Aspergillus, Penicillium, Mucor, and Rhizopus. Cells of a microorganism separated from the culture broth are extracted with an acid such as perchloric acid, hydrochloric acid, sulfuric acid, formic acid, and acetic acid, or an alkylformate to obtain an extract containing SAM. The extract is applied to purification procedures hereinafter provided.

2.    SAM is also synthesized enzymatically from adenosine triphosphate and methionine to obtain a solution containing SAM [S. Hervey Mudd et al., J. Biol. Chem., 231, 481(1958); Japanese Patent publication No. 35727/1977].
The solution containing SAM is applied to the following purification procedures.

B.    Purification of SAM

Isolation of SAM from the solutions containing SAM described above and purification thereof are carried out as follows:
For example, any one of the following procedures or its proper combination can be employed. These procedures include, cation exchange chromatography; fractional precipitation with Reinecke's salt, picric acid, phosphotungstic acid, picrolonic acid, or methyl orange; activated charcoal adsorption chromatography; chelate resin chromatography; and fractional precipitation with polar organic solvents.

C.  The composition of SAM of the present invention and the process for preparation thereof

As described previously, the composition of SAM of the present invention comprises a salt of SAM and lactose and/or maltose. Examples of the salt of SAM are described hereinbefore. Usually, however, various salts of SAM in which the molar ratio of the acid component to SAM is varied are obtained according to the kind of the methods for preparation or conditions therefore.

The salt of SAM in the composition of the present invention consists of SAM and preferably 0.5 - 4 moles, more preferably 2 - 3 moles, of the acid component based on 1 mole of the SAM component.

In the composition, the molar ratio of lactose and/or maltose to SAM is preferably 10 or less, more preferably 0.5 - 5.

The present inventors have found that some disaccharises which have glucose in the structure as their reduced terminal stabilize SAM against its chemical degradation.  Examples of the disaccharide are cellobiose, gentiobiose, lactose, maltose, and melibiose.  Of these disaccharides, lactose and maltose are more effective in stabilizing SAM than others.  Either or both of lactose and maltose can be chosen for incorporation in the composition of the present invention to serve the purpose of the composition in use.  Lactose and maltose in the composition of the present invention are preferably anhydrous and the moisture content of the composition of the present invention must be 3% or less, preferably 1% or less.

The composition of the present invention is stable especially in formulas prescribed above.

For preparation of the composition of the present invention an aqueous solution containing said two components of the composition, that is, a salt of SAM and lactose and/or maltose, is prepared first. In this case, if the molar ratio of acid to SAM in the salt of SAM is less than the value prescribed above, the dificiency of acid is supplied by adding the acid to the aqueous solution of SAM; on the other hand, if said molar ratio is more than the value prescribed above, the excess of the acid in the aqueous solution is removed by an $OH^-$ type anion exchange resin such as Amberlite IR-45 (a trademark of Rhom & Haas Co., U.S.A.) and Dowex-1 (a trademark of The Dow Chemical Co., U.S.A.). The aqueous solution containing a salt of SAM and lactose and/or maltose thus prepared is then lyophilized. Lyophilization is suitable to obtain the composition of the present invention without the degradation of SAM in the composition. As occasion demands, the composition thus obtained may be dried in vacuo over a desiccant such as phosphorus pentoxide and silica gel. In another method for preparing the composition of the present invention a salt of SAM and lactose and/or maltose are mixed in powdery form.

It is desirable that lactose and maltose in the composition of the present invention are anhydrous.

Since commercially available lactose or maltose usually contains one mole water as bound water, its anhydrous type is prepared by dissolving lactose or maltose in an aqueous solvent to prepare its aqueous solution followed by removing the aqueous solvent from the aqueous solution, for example, by lyophilization, drum-drying, or spray-drying.

If, however, anhydride of lactose or maltose is commercially available, it is mixed with a salt of SAM in a powdery state to prepare the composition of the present invention.

The composition of SAM obtained above is dried until it contains 3% or less, preferably 1% or less, of moisture.

The composition of SAM of the present invention is stable for a long period of time even at a room temperature. The stability of the composition of SAM of the present invention shown in examples hereinafter provided was compared with that of four known SAM preparations, that is SAM·chloride (the molar ratio of hydrochloric acid to SAM is 2), SAM·sulfate (the molar ratio of sulfuric acid to SAM is 2), SAM·sulfate (the molar ratio of sulfuric acid to SAM is 2.5), and SAM·sulfate·p-toluenesulfonate (the molar ratios of sulfuric acid and p-toluenesulfonic acid to SAM are 2 and 1, respectively); the results are shown in Table 1.

Table 1

Stability tests of the Compositions
of the Present Invention and Known
SAM Preparations

| | Residual SAM (%) |
|---|---|
| Composition of the present invention (salt of SAM·lactose or maltose) ( ): molar ratio to SAM | |
| SAM·sulfate (2.5)  lactose  (2) | 96.1 |
| SAM·sulfate (2.5)  maltose  (2) | 97.3 |
| SAM·chloride (2)  maltose  (5) | 89.8 |
| SAM·sulfate (2) P-toluenesulfonate (1) lactose (3) | 95.9 |
| SAM·sulfate (2)  maltose (0.86) | 80.3 |
| Known preparation ( ): molar ratio to SAM | |
| SAM·sulfate (2) | 51.4 |
| SAM·sulfate (2.5) | 85.5 |
| SAM·chloride (2) | 18.3 |
| SAM·sulfate (2) P-toluenesulfonate (1) | 87.1 |

Note: The stability of the samples is indicated here by residual SAM after preservation of the samples at 40°C and 75% relative humidity for 6 months in a sealed glass tube.

As will be clear from Table 1, the composition of SAM of the present invention is stable when-stored even at 40°C and 75% relative humidity for 6 months, whereas the four known SAM preparations are not stable enough when stored under these conditions. Thus, whereas the known SAM preparations must be stored at a cool place, the composition of SAM of the present invention can be stored at a room temperature.

To further illustrate the present invention, but not by way of limitation, the following examples are given.

### EXAMPLE 1

A SAM·sulfate consisting of 2 moles of sulfuric acid and 1 mole of SAM was prepared by a known precedure [F. Schlenk et al., Enzymologia, 29, 283 (1965); Japanese patent publication No. 13680/1971]. In 100 ml of distilled water were dissolved 10 g of the SAM·sulfate and 11.9 g of lactose, and to the resulting solution was added 2.8 ml of 6 N sulfuric acid. The resulting aqueous solution was lyophilized at 40°C or less for 36 hours to prepare 21 g of the composition of the present invention. The molar ratio of SAM : sulfuric acid : lactose of the composition was 1 : 2.5 : 2.

### EXAMPLE 2

The composition comprising SAM·sulfate and maltose (21 g) was prepared in the same manner as in Example 1 with the exception that maltose was used in place of lactose.

## EXAMPLE 3

A SAM·chloride consisting of 1.8 moles of hydrochloric acid and 1 mole of SAM was prepared in the same manner as in Example 1 with the exception that hydrochloric acid was used in place of sulfuric acid. In 100 ml of distilled water were dissolved 10 g of the SAM·chloride and 38.1 g of maltose, and to the resulting solution was added 0.78 ml of 6 N hydrochloric acid. The resulting aqueous solution was lyophilized at 40°C or less for 36 hours to prepare 46 g of the composition of the present invention. The molar ratio of SAM : hydrochloric acid : maltose of the composition was 1 : 2 : 5.

## EXAMPLE 4

In 100 ml of distilled water were dissolved 10 g of SAM· sulfate which had been prepared in the same manner as in Example 1, 3.1 g of p-toluenesulfonic acid, and 17.5 g of lactose. In the same manner as in Example 1, 30 g of the composition of the present invention was prepared. The molar ratio of SAM : sulfuric acid : p-toluenesulfonic acid : lactose was 1 : 2 : 1 : 3.

## EXAMPLE 5

In 1 ℓ of distilled water was dissolved 100 g of maltose, and the resulting aqueous solution was lyophilized at 50°C or less for 24 hours to prepare 95 g of anhydrous maltose. The anhydrous maltose (90 g) that had been sieved through a 100 mesh screen was mixed with 90 g of SAM·sulfate that had been prepared in the same manner as in Example 1 by a V-type blender. The resulting mixed powder was dried at 30°C for 3 hours over phosphorus pentoxide under reduced pressure to afford 180 g of the composition of the present invention. The molar ratio of SAM : sulfuric acid : maltose of the composition was 1 : 2 : 0.86.

WHAT IS CLAIMED IS:

1. A stable composition of S-adenosyl-L-methione which comprises a salt of S-adenosyl-L-methionine and lactose and/or maltose.

2. The composition according to Claim 1 wherein the salt of S-adenosyl-L-methionine is a member selected from the group consisting of salts of S-adenosyl-L-methionine with hydrobromic acid, hydrochloric acid, hydroiodic acid, sulfuric acid, and p-toluenesulfonic acid; and a double salt of S-adenosyl-L-methionine with said acids.

3. The composition according to Claim 1 or Claim 2 wherein the salt of S-adenosyl-L-methionine consists of 0.5 - 4 moles of the acid component and 1 mole of the S-adenosyl-L-methionine component.

4. The composition according to Claim 3 wherein the salt of S-adenosyl-L-methionine consists of 2 - 3 moles of the acid component and 1 mole of the S-adenosyl-L-methionine component.

5. The composition according to Claim 1 or Claim 2 wherein the molar ratio of lactose and/or maltose to S-adenosyl-L-methionine is 10 or less.

6. The composition according to Claim 5 wherein the molar ratio of lactose and/or maltose to S-adenosyl-L-methionine is 0.5 - 5.

7. A process for preparation of the composition according to Claim 1 or Claim 2 which comprises dissolving the salt of S-adenosyl-L-methionine and lactose and/or maltose in an aqueous solvent to prepare an aqueous solution of the composition followed by lyophilizing said aqueous solution.

8. A pharmaceutical formulation comprising a composition of claim 1 associated with a pharmaceutically-acceptable carrier therefor.

9. A composition as claimed in any of claims 1 to 7 for use as a pharmaceutical.

European Patent
Office

**EUROPEAN SEARCH REPORT**

Application number

EP 82 11 0230

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| X | US-A-4 028 183 (A.FIECCHI) *Column 15: a,b,* | 1-9 | A 61 K 31/70 |
| | ----- | | |

TECHNICAL FIELDS
SEARCHED (Int. Cl. ³)

A 61 K 31/00
C 07 H 19/16

The present search report has been drawn up for all claims

| Place of search THE HAGUE | Date of completion of the search 24-03-1983 | Examiner VERHULST W. |
|---|---|---|